(19) Europäisches Patentamt
European Patent Office
Office européen des brevets

(11) Veröffentlichungsnummer: **0 203 286**
**B1**

# (12) EUROPÄISCHE PATENTSCHRIFT

(45) Veröffentlichungstag der Patentschrift: **14.09.88**

(51) Int. Cl.[4]: **C 07 C 51/54**, C 07 C 51/56, B 01 J 31/18, B 01 J 31/26

(21) Anmeldenummer: **86103319.9**

(22) Anmeldetag: **12.03.86**

(54) **Trägerkatalysator und Verfahren zur Herstellung von Monocarbonsäureanhydriden.**

(30) Priorität: **27.03.85 DE 3511050**
**27.03.85 DE 3511048**

(43) Veröffentlichungstag der Anmeldung:
**03.12.86 Patentblatt 86/49**

(45) Bekanntmachung des Hinweises auf die Patenterteilung:
**14.09.88 Patentblatt 88/37**

(84) Benannte Vertragsstaaten:
**AT BE CH DE FR GB IT LI NL**

(56) Entgegenhaltungen:
**EP - A - 0 180 799**
**EP - A - 0 180 801**
**EP - A - 0 180 802**
**EP - A - 0 181 502**
**FR - A - 2 242 362**
**GB - A - 2 067 556**

(73) Patentinhaber: **HOECHST AKTIENGESELLSCHAFT, Postfach 80 03 20, D-6230 Frankfurt am Main 80 (DE)**

(72) Erfinder: **Luft, Gerhard, Dr., Ludwigstrasse 141a, D-6109 Mühltal (DE)**
Erfinder: **Ritter, Gebhard, Dr., Johann-Peter-Hebelstrasse 11, D-7601 Schutterwald/Baden (DE)**

**EP 0 203 286 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1-4 C-Atomen bedeutet, mit Kohlenmonoxid in der Gasphase in Gegenwart von Jod oder Brom oder deren Verbindungen als Reaktionspromotor sowie in Gegenwart eines Edelmetallverbindungen aus der Gruppe VIII des Periodensystems enthaltenden Trägerkatalysators bei Temperaturen von 130 bis 400°C und Drücken von 1-150 bar.

Ein derartiges, in der Gasphase mit einem Trägerkatalysator arbeitendes Verfahren ist bereits aus der DE-Offenlegungsschrift 2 450 965 und der JP-Offenlegungsschrift Nr. 47 921/1975 bekannt, welche die bei den Flüssigphase-Verfahren auftretenden Nachteile, z.B. die schwierige Abtrennung und Rückführung von suspendiertem und teilweise gelöstem Katalysator und ggf. Promotor, vermeiden.

In beiden Schriften werden Gasphase-Verfahren mit festen Trägerkatalysatoren beschrieben, welche durch Imprägnierung des Trägermaterials mit gelösten oder suspendierten, auch komplexen, Edelmetallverbindungen hergestellt wurden. Es ist aber auf diese Weise nicht möglich, z.B. Organostickstoff- oder Organophosphorverbindungen mit dreibindigem Stickstoff bzw. Phosphor im Trägerkatalysator zu fixieren, was sich im allgemeinen auf die Katalysatoraktivität und die Selektivität der Reaktion ungünstig auswirkt.

Vorliegende Erfindung vermeidet diesen Mangel durch Imprägnierung des Trägermaterials mit Edelmetall-Chelatverbindungen, in welche Promotoren der V. Hauptgruppe, z.B. Organylamine oder -phosphine, bereits integriert sind.

Im einzelnen ist die Erfindung dadurch gekennzeichnet, dass

1) im Trägerkatalysator das Trägermaterial eine Edelmetall-Chelatverbindung trägt, die aus der Edelmetallverbindung und einem Chelator mit Organostickstoff-, Organophosphor-, Organoarsen- oder Organoschwefelgruppen gebildet ist.

Darüberhinaus kann das Verfahren der Erfindung wahlweise und bevorzugt dadurch gekennzeichnet sein, dass

2) im Trägerkatalysator das Trägermaterial zusätzlich eine Nichtedelmetall-Chelatverbindung trägt, die aus einer Nichtedelmetallverbindung aus der 6. oder 8. Nebengruppe des Periodensystems der Elemente und einem Chelator mit Organostickstoff-, Organophosphor-, Organoarsen- oder Organoschwefelgruppen gebildet ist;

3) der Trägerkatalysator zusätzlich Nichtedelmetallverbindungen aus der 1. bis 3. Hauptgruppe oder der 4. bis 6. oder der 8. Nebengruppe des Periodensystems der Elemente als Promotoren enthält;

4) im Trägerkatalysator das Trägermaterial eine Chelatverbindung trägt, die aus der Metallverbindung und einem der folgenden Chelatoren gebildet ist:

a) $Y-(CH_2)_n-Y$

b) $Y-CH=CH-Y$

c) $Ø_2P-CH=CH-PØ_2$

d) $Ø_2As-CH=CH-AsØ_2$

e) $Ø_2P-CH_2-CH_2-PØ-CH_2-CH_2-PØ-CH_2CH_2-PØ_2$

f) $Ø_2P-CH_2-CH_2-PØ-CH_2-CH_2-PØ_2$

g)

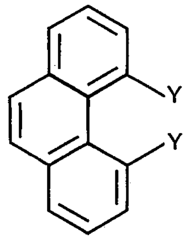

h) $P(-CH_2CH_2-PØ_2)_3$

i) $R^1-C[-(CH_2)_n-Y]_3$

j)

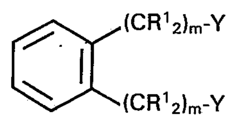

k) $Ø_2P-(CH_2)_x$ $(CH_2)_x-PØ_2$
$Ø_2P-(CH_2)_x$ >N-$CH_2-CH_2$-N< $(CH_2)_x-PØ_2$

wobei

$Ø = C_6H_5$-

Y = $-NR^2_2$, ein stickstoffhaltiger Arylrest, $-PR^2_2$, $-AsR^2_2$, $-SR^2$ oder -SH;

$R^1$ = -H, $C_1$ bis $C_5$-Alkyl oder $-C_6H_5$;

$R^2$ = $C_1$ bis $C_6$-Alkyl, $C_5$ bis $C_8$ Cycloalkyl oder $-C_6H_5$ oder $C_6H_5CH_2$-, die ggf. mit Halogen-, Methoxy-, Ethoxy- oder $C_1$ bis $C_3$-Alkyl substituiert sind;

n = 1-6, vorzugsweise 1-4;

m = 0-8, vorzugsweise 0-3;

x = 1 oder 2;

5) der Trägerkatalysator ein anorganisches oxidisches Trägermaterial oder einen Aktivkohleträger enthält;

6) der Trägerkatalysator zusammen 0,01 bis 50 Gew%, vorzugsweise 0,1 bis 20 Gew%, Chelatverbindungen und ggf. Nichtedelmetallverbindungen enthält;

7) der Trägerkatalysator in einer Korngrösse von 1 bis 20 mm eingesetzt wird.

Die Erfindung betrifft ebenso auch den Trägerkatalysator selbst für die Herstellung von Monocarbonsäureanhydriden durch Carbonylierung der entsprechenden Ester oder Esther, welche dadurch gekennzeichnet ist, dass in ihm das Trägermaterial eine Edelmetall-Chelatverbindung trägt, die aus einer Edelmetallverbindung der 8. Nebengruppe des Periodensystems der Elemente und einem Chelator mit Organostickstoff-, Organophosphor-, Organoarsen- oder Organoschwefelgruppen gebildet ist.

Der Trägerkatalysator der Erfindung kann weiterhin wahlweise und bevorzugt dadurch gekennzeichnet sein, dass

1) in ihm das Trägermaterial zusätzlich eine Nichtedelmetall-Chelatverbindung trägt, die aus einer Nichtedelmetallverbindung aus der 6. oder 8. Nebengruppe des Periodensystems der Elemente und einem Chelator mit Organostickstoff-, Organophosphor-, Organoarsen- oder Organoschwefelgruppen gebildet ist;

2) er zusätzlich Nichtedelmetallverbindungen aus der 1. bis 3. Hauptgruppe oder der 4. bis 6. oder der 8. Nebengruppe des Periodensystems der Elemente als Promotoren enthält;

3) in ihm das Trägermaterial eine Chelatverbindung trägt, die aus der Metallverbindung und einem der oben unter 4) a) bis k) aufgezählten Chelatoren gebildet ist;

4) er ein anorganisches oxidisches Trägermaterial oder einen Aktivkohleträger enthält;

5) er zusammen 0,01 bis 50 Gew.-%, vorzugsweise 0,1 bis 20 Gew.-%, Chelatverbindungen und ggf. Nichtedelmetallverbindungen enthält.

Als Katalysatorträger kommen bevorzugt anorganische Oxide wie z.B. $SiO_2$, $Al_2O_3$, MgO, $TiO_2$, $La_2O_3$, $ZrO_2$, Zeolith, Ton, NiO, $Cr_2O_3$, $WO_3$ oder entsprechende Mischoxide, aber auch Aktivkohle infrage, welche BET-Oberflächen von 1 - 1000 $m^2/g$, vorzugsweise 30 - 400 $m^2/g$, haben.

Die Promotoren der 5. oder 6. Hauptgruppe sind in den erfindungsgemäss eingesetzten Chelatoren chemisch gebunden und bilden selbst eine ihrer funktionellen Gruppen, welche die Edelmetallverbindungen aus der Gruppe VIII, insbesondere Rh, Ir, Pd oder Ru, und ggf. Nichtedelmetallverbindungen aus der 6. oder 8. Nebengruppe, insbesondere Cr oder Ni, aber auch W, Fe und Co, krebsscherenartig umschliessen.

Es ist ein Vorteil des Trägerkatalysators und des Verfahrens der Erfindung, dass die zur Steigerung der Katalysatoraktivität und der Selektivität notwendigen Promotoren aus der Hauptgruppe V oder VI des Periodensystems der Elemente eine funktionelle Gruppe Y in den Chelatoren bilden und somit fixiert sind. Deshalb entfällt eine Abtrennung und Rückführung dieser z.B. Organostickstoff- oder Organophosphorpromotoren. Das Verfahren der Erfindung zur Herstellung von Monocarbonsäureanhydriden weist gegenüber den eingangs beschriebenen bekannten Verfahren, welche bereits in der Gasphase mit einem Trägerkatalysator arbeiten, höhere Katalysatoraktivitäten und Selektivitäten auf.

Ein weiterer Vorteil der Erfindung ist darin zu sehen, dass die auf dem Trägermaterial aufgetragenen Edelmetall-Chelatverbindungen und ggf. Nichtedelmetall-Chelatverbindungen bei den für die Herstellung von Monocarbonsäureanhydriden erforderlichen Reaktionstemperaturen noch nicht schmelzen.

Der Trägerkatalysator und das Verfahren der Erfindung dienen insbesondere zur Herstellung von Essigsäureanhydrid aus Methylacetat oder Dimethylether in Gegenwart von Methyljodid oder Methylbromid als Reaktionspromotor. Als Reaktionspromotor kann auch HJ, HBr ode allgemein RJ oder RBr eingesetzt werden, wobei R einen Alkylrest mit 1-4 C-Atomen bedeutet.

Die Trägermaterialien wurden bereits genannt; als Mischoxide kommen z.B. $Cr_2O_3$ - $Al_2O_3$, $WO_3$ - $Al_2O_3$, MgO - $Al_2O_3$, $SiO_2$ - $Al_2O_3$ oder $ZrO_2$ - $Al_2O_3$ infrage. Der Trägerkatalysator enthält bevorzugt 0,01 bis 5 Gew.-% Edelmetall und wird in einer Korngrösse von 1 bis 20 mm eingesetzt.

Als Edelmetallverbindungen kommen bei der Herstellung des Trägerkatalysators z.B. folgende Verbindungen infrage:

Rhodium:

$RhCl_3$, $RhCl_3 \cdot 3\,H_2O$, $RhBr_3$, $RhJ_3$, $Rh(NO_3)_3$, $Rh_2(CO)_4,Cl_2$, $Rh_2(CO)_4Br_2$, $Rh(CO)_4J_2$, $[P(C_6H_5)_3]_3RhCl$, $[P(C_6H_5)_3]_2\,Rh(CO)Cl$, $Rh_6(CO)_{16}$, $Rh_4(CO)_{12}$, $Rh_2(O_2CCH_3)_4$, $[RhCl(C_8H_{12})]_2$;

Iridium:

$IrCl_3$, $[Ir(CO)_3Cl]_2$, $Ir[P(C_6H_5)_3]_2(CO)Cl$, $Ir_4(CO)_{12}$, $[IrCl(C_8H_{12})]_2$, $Cl(CO)_2Jrpyr$ (pyr = $C_6H_5N$);

Palladium:

$PdCl_2$, $PdBr_2$, $PdI_2$, $(CH_3CO_2)_2Pd[P(C_6H_5)_3]_2$, $PdCl_2[P(C_6H_5)_3]_2$, $Pd(O_2CCH_3)_2$, $PdCl_2(C_8H_{12})$, $(C_6H_5CN)_2PdCl_2$;

Ruthenium:

$RuCl_3$, $Ru_3(CO)_{12}$, $RuCl_2[P(C_6H_5)_3]_3$, $RuCl_2(CO)_2[P(C_6H_5)_3]_2$, $[RuCl_2(CO)_3]_2$.

Als Nichtedelmetallverbindungen aus der 6. oder 8. Nebengruppe, insbesondere Cr, Ni, aber auch W, Fe, Co, die ebenfalls mit den Chelatoren reagieren, kommen z.B. ferner infrage:

Chrom:

$Cr(CO)_6$, $CrCl_3$, $C_7H_8Cr(CO)_3$.

Nickel:

$Ni(CO)_4$, $[P(C_6H_5)_3]_2Ni(CO)_2$, $NiCl_2$, $Ni(C_8H_{12})_2$.

Als Nichtedelmetallverbindungen aus der 1. bis 3. Hauptgruppe oder der 4. bis 6. oder der 8. Nebengruppe des Periodensystems, vorzugsweise des Li, Na, Mg, Ca, Al, Ti, Zr, V, Cr, W, Fe, Co, Ni, können z.B. Hydroxide, Carbonate, Carbonyle, Hydride, Halogenide und andere Salze eingesetzt werden. Diese Verbindungen unedler Metalle können zusätzlich z.B. als Lösung durch Imprägnierung auf den Katalysatorträger aufgebracht werden.

Zur Herstellung des erfindungsgemässen Trägerkatalysators muss zuerst der Chelator mit den funktionellen Gruppen Y bereitgestellt werden. Dieser ist entweder im Handel erhältlich oder kann nach oder in Analogie zu Literaturangaben dargestellt werden. Im allgemeinen wird dann eine der genannten Edelmetallverbindungen aus der Gruppe VIII und ggf. eine der genannten Nichtedelmetallverbindungen aus der 6. oder 8. Nebengruppe in Lösung mit dem Chelator in Verbindung gebracht, wobei in an sich bekannter Weise bekannte Chelatverbindungen entstehen, deren Schmelzpunkte über der Temperatur der Carbonylierungsreaktion zur Herstellung von Monocarbonsäureanhydriden liegen. Anschliessend erfolgt die Imprägnierung des Trägermaterials mit den gelösten bekannten Chelatverbindungen zum fertigen Trägerkatalysator. Die Lösemittel für die Chelatverbindungen können polar und unpolar sein, z.B. Dichlormethan (Methylenchlorid), Chloroform, Methanol, Benzol, Toluol oder Xylol, worin das Trägermaterial suspendiert wird.

Alle Einzelheiten ergeben sich aus der Katalysatorbeschreibung.

Das Mengenverhältnis von Carbonsäureester bzw. Dialkylether und Jod(verbindung) oder Brom(verbindung) in der Reaktionszone kann innerhalb weiter Grenzen schwanken. Im allgemeinen beträgt die Menge des Carbonsäureesters und/oder Dialkylethers 1 bis 500 Mol, vorzugsweise 1 bis 100 Mol, je 1 Mol Jod(verbindung) oder Brom(verbindung). Die Temperatur der Reaktionszone wird so gewählt, dass das Reaktionsgemisch bei jedem beliebigen Umsatz gasförmig vorliegt. Bevorzugt wird die Temperatur zwischen 150 und 250°C gewählt. Der bevorzugte Druck liegt zwischen 5 und 30 bar.

Die Verweilzeit des Reaktionsgemisches am festen Trägerkatalysator beträgt 1 bis 1000 s, bevorzugt 1 bis 180 s. Die Umsetzung kann in einem vorzugsweise senkrecht angeordneten und mit Trägerkatalysator gefüllten Strömungsrohr oder auch in einem Rühr- oder Schüttelautoklaven erfolgen, der den Trägerkatalysator enthält. Man führt die Carbonylierung im allgemeinen unter praktisch wasserfreien Bedingungen durch, doch ist die Anwesenheit geringer Wassermengen, wie sie in den handelsüblichen Ausgangsstoffen vorkommen, zulässig, sollte aber 1 Mol%, berechnet auf die Ausgangsstoffe, nicht übersteigen. Auch wird die Carbonylierung durch geringe Mengen Methanol in den Ausgangsstoffn nicht beeinträchtigt. Ebensowenig stört Wasserstoff, der in kleinen Mengen im handelsüblichen Kohlenmonoxid vorhanden sein kann.

Das aus der Carbonylierungszone abströmende Reaktionsgemisch ist gasförmig und enthält Kohlenmonoxid, Methyljodid, Essigsäureanhydrid, nichtumgesetztes Methylacetat oder Dimethylether und ggf. geringe Mengen Essigsäure. Das gasförmige Reaktionsgemisch wird abgekühlt, wobei Essigsäureanhydrid und ggf. Essigsäure auskondensieren. Die nicht kondensierten Gase wie CO, $CH_3J$, Methylacetat oder Dimethylether werden in die Reaktionszone zurückgeführt, wobei die umgesetzten Anteile von Ester oder Esther sowie CO fortlaufend ersetzt werden. Die einfache Abtrennung der Anhydride durch Kühlung des abströmenden Reaktionsgemisches und Rückführung der nicht kondensierbaren Gase stellt einen wesentlichen Vorteil des erfindungsgemässen Verfahrens dar, da dies ohne komplizierte Trennoperationen erfolgen kann. Der Trägerkatalysator wird nicht verunreinigt und verbleibt in der Reaktionszone, was den gesamten Verfahrensablauf bedeutend vereinfacht.

*Beispiele*

Druckautoklavversuche

Man verwendet einen 0,25 Liter fassenden Rührautoklav aus korrosionsfreiem Edelstahl (Hastelloy C) mit den erforderlichen Zu- und Ableitungen, der einen drehbaren Katalysatorkorb enthält. Die Carbonsäureester oder Dialkylether werden gasförmig in Gegenwart des bewegten, festen Trägerkatalysators mit CO-Gas umgesetzt. Der Trägerkatalysator befindet sich in dem drehbaren Katalysatorkorb, der gleichzeitig für die Durchmischung der Gase sorgt. Der Autoklav wird mit 2,5 ml eines flüssigen Gemisches aus 20 Volumenteilen Methyljodid und 80 Volumenteilen Ester oder Ether beschickt und auf Reaktionstemperatur aufgeheizt. Die Carbonylierung wird durch Aufpressen von Kohlenmonoxid eingeleitet. Der CO-Druck wird durch regelmässiges Nachdrücken konstant gehalten. Die Einzelheiten ergeben sich aus den einzelnen Beispielen.

*Beispiel 1*

2 ml (1,86 g) Essigsäuremethylester, 0,5 ml (1,14 g) Methyljodid und 1,60 g Katalysator Nr. 1 werden im Autoklaven mit Kohlenmonoxid bei 20 bar CO-Druck und 180°C umgesetzt. Nach 1 h Reaktionszeit ergibt sich eine Katalysatorleistung von 260 g $Ac_2O/g_{Rh} \cdot h$. Die Ausbeute von $Ac_2O$, bezogen auf den eingesetzten Ester, beträgt 64 % bei einer Selektivität von 95%.

*Beispiel 2*

2 ml (1,86 g) Essigsäuremethylester 0,5 ml (1,14 g) Methyljodid und 1,60 g Katalysator Nr. 1 werden im Autoklaven mit Kohlenmonoxid bei 20 bar CO-Druck und 175°C umgesetzt. Nach 1 h Reaktionszeit ergibt sich eine Katalysatorleistung von 220 g $Ac_2O/g_{Rh} \cdot h$. Die Ausbeute von $Ac_2O$ bezogen auf den eingesetzten Ester, beträgt 54% bei einer Selektivität von 96%.

*Beispiel 3*

2 ml (1,86 g) Essigsäuremethylester, 0,5 ml (1,14 g) Methyljodid und 1,77 g Katalysator Nr. 2 werden im Autoklaven mit Kohlenmonoxid bei 20 bar CO-Druck und 166°C umgesetzt. Nach 1 h Reaktionszeit ergibt sich eine Katalysatorleistung von 280 g $Ac_2O/g_{Rh} \cdot h$. Die Ausbeute von $Ac_2O$, bezogen auf den eingesetzten Ester, beträgt 64% bei einer Selektivität von 97%.

*Beispiel 4*

2 ml (1,86 g) Essigsäuremethylester, 0,5 ml (1,14 g) Methyljodid und 1,77 g Katalysator Nr. 2 werden im Autoklaven mit Kohlenmonoxid bei 20 bar CO-Druck und 180°C umgesetzt. Nach 1 h Reaktionszeit ergibt sich eine Katalysatorleistung von 380 g $Ac_2O/g_{Rh} \cdot h$. Die Ausbeute von $Ac_2O$, bezogen auf den eingesetzten Ester, beträgt 86% bei einer Selektivität von 93%.

*Beispiel 5*

2 ml (1,86 g) Essigsäuremethylester, 0,5 ml (1,14 g) Methyljodid und 1,78 g Katalysator Nr. 3 werden im Autoklaven mit Kohlenmonoxid bei 20 bar CO-Druck und 200°C umgesetzt. Nach 1 h Reaktionszeit ergibt sich eine Katalysatorleistung von 35 g $Ac_2O/g_{Rh} \cdot h$. Die Ausbeute von $Ac_2O$, bezogen auf den eingesetzten Ester, beträgt 11,6% bei einer Selektivität von 87%.

*Beispiel 6*

2 ml (1,86 g) Essigsäuremethylester, 0,5 ml (1,14 g) Methyljodid und 1,70 g Katalysator Nr. 4 werden

im Autoklaven mit Kohlenmonoxid bei 20 bar CO-Druck und 180°C umgesetzt. Nach 1 h Reaktionszeit ergibt sich eine Katalysatorleistung von 450 g $Ac_2O/g_{Rh} \cdot h$. Die Ausbeute von $Ac_2O$, bezogen auf den eingesetzten Ester, beträgt 24% bei einer Selektivität von 94,7%.

*Beispiel 7*

2 ml (1,86 g) Essigsäuremethylester, 0,5 ml (1,14 g) Methyljodid und 4,4 g Katalysator Nr. 5 werden im Autoklaven mit Kohlenmonoxid bei 20 bar CO-Druck und 180°C umgesetzt. Nach 1 h Reaktionszeit ergibt sich eine Katalysatorleistung von 150 g $Ac_2O/g_{Rh} \cdot h$. Die Ausbeute von $Ac_2O$, bezogen auf den eingesetzten Ester, beträgt 78% bei einer Selektivität von 94%.

*Beispiel 8*

2 ml (1,86 g) Essigsäuremethylester, 0,5 ml (1,14 g) Methyljodid und 1,7 g Katalysator Nr. 6 werden im Autoklaven mit Kohlenmonoxid bei 20 bar CO-Druck und 180°C umgesetzt. Nach 1 h Reaktionszeit ergibt sich eine Katalysatorleistung von 190 g $Ac_2O/g_{Rh} \cdot h$. Die Ausbeute von $Ac_2O$, bezogen auf den eingesetzten Ester, beträgt 45% bei einer Selektivität von 93%.

*Beispiel 9*

Ein Stahlrohr von 20 mm Durchmesser und 450 mm Länge wird als Strömungsrohr senkrecht angeordnet und mit 27,4 g Katalysator analog Nr. 2 gefüllt, dessen Rh-Gehalt jedoch 0,4 Gew.-% betrug. Bei einem Druck von 12,5 bar und 172°C werden stündlich 11 Nl CO (Nl = Liter, gemessen bei 1,013 bar und 0°C) sowie ein verdampftes Gemisch (13 ml Flüssigkeit) aus Methylacetat und Methyljodid (Molverhältnis 11 : 1) durch das Strömungsrohr geleitet.

Das abströmende Reaktionsgemisch wird unter Normaldruck auf 0°C abgekühlt und gaschromatographisch analysiert. Hierbei ergibt sich eine Raum-Zeit-Ausbeute von 71 g $Ac_2O/l \cdot h$. Die Ausbeute von $Ac_2O$, bezogen auf den eingesetzten Ester, beträgt 30% bei einer Selektivität von 96%.

Unter diesen Reaktionsbedingungen wurde die Carbonylierung 100 Stunden durchgeführt, wobei der eingesetzte Trägerkatalysator keinen Aktivitätsverlust zeigte.

*Beispiel 10*

2 ml (1,86 g) Essigsäuremethylester, 0,5 ml (1,14 g) Methyljodid und 1,7 g Katalysator Nr. 7 werden im Autoklaven mit Kohlenmonoxid bei 20 bar CO-Druck und 180°C umgesetzt. Nach 1 h Reaktionszeit ergibt sich eine Katalysatorleistung von 300 g $Ac_2O/g_{Rh} \cdot h$. Die Ausbeute von $Ac_2O$ bezogen auf den eingesetzten Ester beträgt 62% bei einer Selektivität von 95%.

*Beispiel 11*

1,86 g Dimethylether, 0,5 ml (1,14 g) Methyljodid und 1,7 g Katalysator Nr. 7 werden im Autoklaven mit Kohlenmonoxid bei 20 bar CO-Druck und 180°C umgesetzt. Nach 1 h Reaktionszeit ergibt sich eine Katalysatorleistung von 100 g $Ac_2O/g_{Rh} \cdot h$.

Die Ausbeute von $Ac_2O$, bezogen auf den eingesetzten Ether, beträgt 20,6% bei einer Selektivität von 85%.

*Beispiel 12*

2 ml (1,86 g) Essigsäuremethylester, 0,5 ml (1,14 g) Methyljodid und 1,7 g Katalysator Nr. 8 werden im Autoklaven mit Kohlenmonoxid bei 20 bar CO-Druck und 180°C umgesetzt. Nach 1 h Reaktionszeit ergibt sich eine Katalysatorleistung von 243 g $Ac_2O/g_{Rh} \cdot h$.

Die Ausbeute von $Ac_2O$, bezogen auf den eingesetzten Ester, beträgt 50,1% bei einer Selektivität von 94%.

*Beispiel 13*

2 ml (1,86 g) Essigsäuremethylester, 0,5 ml (1,14 g) Methyljodid und 1,7 g Katalysator Nr. 9 werden im Autoklaven mit Kohlenmonoxid bei 20 bar CO-Druck und 180°C umgesetzt. Nach 1 h Reaktionszeit ergibt sich eine Katalysatorleistung von 250 g $Ac_2O/g_{Rh} \cdot h$.

Die Ausbeute von $Ac_2O$, bezogen auf den eingesetzten Ester, beträgt 55,0% bei einer Selektivität von 95,5%.

Beschreibung der Katalysatorherstellung

In allen Fällen wurden die Katalysatorträger zwecks Aktivierung zuvor bei 200°C und etwa 0,133 mbar 10 h getrocknet. Alle Synthesen wurden in Gegenwart von Stickstoff unter Ausschluss von Sauerstoff und Wasser durchgeführt, wobei sämtliche Reagenzien zuvor über Molekularsieb 4 A getrocknet worden waren.

Folgende Abkürzungen werden verwendet:

Ø = $C_6H_5$-

dpe = $Ø_2P\text{-}CH_2CH_2\text{-}PØ_2$

dpen = $Ø_2P\text{-}CH{=}CH\text{-}PØ_2$

dpb = $Ø_2P\text{-}(CH_2)_4\text{-}PØ_2$

Tetraphos-1 = $Ø_2PCH_2CH_2PØCH_2CH_2PØCH_2CH_2PØ_2$

Katalysator Nr. 1

$Al_2O_3$ ] $[Rh(dpe)_2]^+Cl^-$

3 g aktivierte Aluminiumoxidkugeln (99% $Al_2O_3$) mit einem Durchmesser von 3 mm, einer inneren Oberfläche nach BET von 125 $m^2/g$ und einem Porenvolumen von 0,9 ml/g wurden zu 150 mg (16 mg Rh) der Verbindung $[Rh(dpe)_2]Cl$, [F.P. = 217°C; hergestellt aus 1,2-Bis-(diphenylposphin)ethan und Dichlorotetracarbonyldirhodium, vgl. A. Sacco et al., J. Chem. Soc. (London), (1964), 3274; zur Herstellung von $[Rh(CO)_2Cl]_2$ aus $RhCl_3 \cdot 3\ H_2O$ und CO-Gas siehe J.A. McCleverty et al., Inorg. Synth. 8 (1966) S. 211; zur Herstellung von $Ø_2PCH_2CH_2PØ_2$ siehe W. Hewertson et al., J. Chem. Soc. (London), (1962), 1490], gelöst in 100 ml Dichlormethan, unter $N_2$-Atmosphäre hinzugegeben.

Die gelbe Suspension wurde unter Rühren bis zum Sieden erhitzt und 12 h unter Rückfluss gehalten, wobei sich das Dichlormethan vollständig entfärbte. Danach wurde das Dichlormethan unter vermindertem Druck abgezogen und der Katalysator bei 1,13 mbar und 85°C acht Stunden getrocknet.

Charakterisierung: Gelbe Pellets
Rh-Gehalt: 0,44 Gew.-%

Katalysator Nr. 2

$Al_2O_3$ ] $[Rh(dpe)_2]^+BF_4^-$

3 g aktivierte Aluminiumoxidkugeln (99% $Al_2O_3$) mit einem Durchmesser von 3 mm, einer inneren Oberfläche nach BET von 125 m²/g und einem Porenvolumen von 0,9 ml/g wurden zu 100 mg (10,4 mg Rh) der Verbindung $[Rh(dpe)_2]BF_4$, [F.P. = 270°C; hergestellt analog Katalysator 1 unter zusätzlichem Anionenaustausch mit $AgBF_4$ zur Erhöhung der Aktivität nach B.R. James et al., Can. J. Chem. 57, 180 (1979)], gelöst in 100 ml Dichlormethan, unter $N_2$-Atmosphäre hinzugegeben. Die gelbe Suspension wurde unter Rühren bis zum Sieden erhitzt und 12 h unter Rückfluss gehalten, wobei sich das Dichlormethan vollständig entfärbte. Danach wurde das Dichlormethan unter vermindertem Druck abgezogen und der Katalysator bei 1,13 mbar und 85°C acht Stunden getrocknet.
Charakterisierung: Gelbe Pellets
Rh-Gehalt: 0,32 Gew.-%

Katalysator Nr. 3

$SiO_2$ ] $[Rh(dpe)_2]^+BF_4^-$

4 g aktiviertes Siliciumdioxid (98% $SiO_2$) mit einem Durchmesser von 3 mm, einer inneren Oberfläche nach BET von 280 m²/g und einem Porenvolumen von 0,95 ml/g wurden zu 193 mg (20,1 mg Rh) der Verbindung $[Rh(dpe)_2]BF_4$, gelöst in 100 ml Dichlormethan, unter $N_2$-Atmosphäre hinzugegeben. Die gelbe Suspension wurde unter Rühren bis zum Sieden erhitzt und 12 h unter Rückfluss gehalten, wobei sich das Dichlormethan vollständig entfärbte. Danach wurde das Dichlormethan unter vermindertem Druck abgezogen und der Katalysator bei 1,13 mbar und 85°C acht Stunden getrocknet.
Charakterisierung: Gelbe Pellets
Rh-Gehalt: 0,47 Gew.-%

Katalysator Nr. 4

$Al_2O_3$ ] $[Rh(dpb)(CO)Cl]_2$

5,3 g aktivierte Aluminiumoxidkugeln (99% $Al_2O_3$) mit einem Durchmesser von 3 mm, einer inneren Oberfläche nach BET von 125 m²/g und einem Porenvolumen von 0,9 ml/g wurden zu 29 mg (5,04 mg Rh) der Verbindung $[Rh(dpb)(CO)Cl]_2$ [F.P. = 182°C; hergestellt aus 1,4-Bis-(diphenylphosphin)butan und Dichlorotetracarbonyldirhodium, vgl. A.R. Sanger, J. Chem. Soc. Dalton Trans. (1977) 120], gelöst in 50 ml Dichlormethan, unter $N_2$-Atmosphäre hinzugegeben. Die gelbe Suspension wurde unter Rühren bis zum Sieden erhitzt und 18 Stunden unter Rückfluss gehalten, wobei sich das Lösemittel Dichlormethan vollständig entfärbte. Danach wurde das Dichlormethan unter vermindertem Druck abgezogen und anschliessend der Katalysator bei 1,13 mbar und 85°C acht Stunden getrocknet.
Charakterisierung: Gelbe Pellets
Rh-Gehalt: 0,08 Gew.-%

Katalysator Nr. 5

$Cr_2O_3$
$Al_2O_3$ ] $[Rh(dpe)_2]^+BF_4^-$

6,3 g aktivierte Chrom-Aluminiumoxidzylinder (5,29 g $Al_2O_3$ + 1,01 g $Cr_2O_3$) mit den Abmessungen 4 × 4 mm und einer inneren Oberfläche nach BET von 68 m²/g wurden zu 200 mg (20,9 mg Rh) der Verbindung $[Rh(dpe)_2]BF_4$, gelöst in 100 ml Dichlormethan, unter $N_2$-Atmosphäre hinzugegeben. Die grüne Suspension wurde unter Rühren bis zum Sieden erhitzt und 24 Stunden unter Rückfluss gehalten, wobei sich das Dichlormethan vollständig entfärbte. Anschliessend wurde das Dichlormethan unter vermindertem Druck abgezogen und der Katalysator bei 1,13 mbar und 85°C acht Stunden getrocknet.
Charakterisierung: Grüne Pellets
Rh-Gehalt: 0,3 Gew.-%

Katalysator Nr. 6

$Al_2O_3$ ] $[Rh(Tetraphos\ I)]^+PF_6^-$

3,5 g aktivierte Aluminiumoxidkugeln (99% $Al_2O_3$) mit einem Durchmesser von 3 mm, einer inneren Oberfläche nach BET von 125 m²/g und einem Porenvolumen von 0,9 ml/g wurden zu 100 ml (11 mg Rh) der Verbindung $[Rh(Tetraphos\text{-}1)]^+PF_6^-$, [F.P. = 314°C; hergestellt aus $(P\emptyset_3)RhCl$ und Tetraphos-1 nach R.B. King et al., Inorg. Chem. Vol. 10, (1971) S. 1851 ff.], gelöst in 50 ml Dichlormethan, unter $N_2$-Atmosphäre hinzugegeben. Die gelbe Suspension wurde unter Rühren bis zum Sieden erhitzt und 16 h unter Rückfluss gehalten, wobei sich das Dichlormethan vollständig entfärbte. Danach wurde das Dichlormethan unter vermindertem Druck abgezogen und der Katalysator bei 1,13 mbar und 85°C acht Stunden getrocknet.
Charakterisierung: Gelbe Pellets
Rh-Gehalt: 0,3 Gew.-%

Katalysator Nr. 7

$Al_2O_3$ ] $[Rh(dpe)_2]^{\oplus}BF_4^{\ominus}$
$[Cr(dpe)(CO)_4]$

3 g aktivierte Aluminiumoxidkugeln (99% $Al_2O_3$) mit einem Durchmesser von 3 mm, einer inneren Oberfläche nach BET von 125 m²/g und einem Porenvolumen von 0,9 ml/g wurden zu 100 mg (10,4 mg Rh) der Verbindung $[Rh(dpe)_2]BF_4$ und 100 g (9,25 mg Cr) der Verbindung $[Cr(dpe)(CO)_4]$, hergestellt nach J. Chatt et al., J. Chem. Soc. (London) 1961, S. 4980 ff, gelöst in 100 ml Dichlormethan, unter $N_2$-Atmosphäre hinzugegeben. Die gelbe Suspension wurde unter Rühren bis zum Sieden erhitzt und 12 h unter Rückfluss gehalten, wobei sich das Dichlormethan vollständig entfärbte. Danach wurde das Dichlormethan unter vermindertem Druck abgezogen und der Katalysator bei 1,13 mbar und 85°C acht Stunden getrocknet.
Charakterisierung: Gelbe Pellets
Rh-Gehalt: 0,31 Gew.-%
Cr-Gehalt: 0,28 Gew.-%

Katalysator Nr. 8

$Al_2O_3$ ] $[Rh(dpe)_2]^{\oplus}BF_4^{\ominus}$ / NaJ

Zu 3 g aktivierten Aluminiumoxidkugeln (99% $Al_2O_3$) mit einem Korndurchmesser von 3 mm, einer inneren Oberfläche nach BET von 125 m²/g und einem Porenvolumen von 0,9 ml/g gab man 0,1 g Natriumjodid, gelöst n 30 ml Aceton, unter Rühren hinzu und erhitzte bis zum Sieden. Nach 48 Stunden Rückfluss wurden das Lösemittel abgezogen und die Katalysatorkugeln bei 1,13 mbar und 85°C acht Stunden getrocknet.

Die Rhodiumkomponente wurde analog Katalysator Nr. 2 aufgebracht.

| | |
|---|---|
| Charakterisierung: | Gelbe Pellets |
| Rh-Gehalt: | 0,31 Gew.-% |
| NaJ-Gehalt: | 3,12 Gew.-% |

Katalysator Nr. 9

$Al_2O_3$ ] $[Rh(dpen)_2]^{\oplus}ClO_4^{\ominus}$

Zu 3 g aktivierten Aluminiumoxidkugeln (99% $Al_2O_3$) mit einem Korndurchmesser von 3 mm, einer inneren Oberfläche nach BET von 125m²/g und einem Porenvolumen von 0,9 ml/g gab man 100 mg (10,3 mg Rh) der Verbindung $[Rh(dpen)_2]^{\oplus}ClO_4^{\ominus}$, hergestellt nach W.A. Fordyce et al., Inorg. Chem. 1982, 21, S. 1455-61, gelöst in 100 ml Dichlormethan, unter $N_2$-Atmosphäre hinzu. Die hellgelbe Suspension wurde bis zum Sieden erhitzt und 12 h unter Rückfluss gehalten, wobei sich das Lösemittel vollständig entfärbte. Danach wurde das Lösemittel unter vermindertem Druck abgezogen und der Katalysator bei 1,13 mbar und 85°C acht Stunden getrocknet.

| | |
|---|---|
| Charakterisierung: | Gelbe Pellets |
| Rh-Gehalt: | 0,33 Gew.-% |

**Patentansprüche**

1. Verfahren zur Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1-4 C-Atomen bedeutet, mit Kohlenmonoxid in der Gasphase in Gegenwart von Jod oder Brom oder deren Verbindungen als Reaktionspromotor sowie in Gegenwart eines Edelmetallverbindungen aus der Gruppe VIII des Periodensystems enthaltenden Trägerkatalysators bei Temperaturen von 130 bis 400°C und Drücken von 1-150 bar, dadurch gekennzeichnet, dass im Trägerkatalysator das Trägermaterial eine Edelmetall-Chelatverbindung trägt, die aus der Edelmetallverbindung und einem Chelator mit Organostickstoff-, Organophosphor-, Organoarsen- oder Organoschwefelgruppen gebildet ist.

2. Verfahren nach Anspruch 1, dadurch gekennzeichnet, dass im Trägerkatalysator das Trägermaterial zusätzlich eine Nichtedelmetall-Chelatverbindung trägt, die aus einer Nichtmetallverbindung aus der 6. oder 8. Nebengruppe des Periodensystems der Elemente und einem Chelator mit Organostickstoff-, Organophosphor-, Organoarsen- oder Organoschwefelgruppen gebildet ist.

3. Verfahren nach Anspruch 1 oder 2, dadurch gekennzeichnet, dass der Trägerkatalysator zusätzlich Nichtedelmetallverbindungen aus der 1. bis 3. Hauptgruppe oder der 4. bis 6. oder der 8. Nebengruppe des Periodensystems der Elemente als Promotoren enthält.

4. Verfahren nach einem der Ansprüche 1 bis 3, dadurch gekennzeichnet, dass im Trägerkatalysator das Trägermaterial eine Chelatverbindung trägt, die aus der Metallverbindung und einem der folgenden Chelatoren gebildet ist:

a) $Y\text{-}(CH_2)_n\text{-}Y$

b) $Y\text{-}CH=CH\text{-}Y$

c) $\emptyset_2P\text{-}CH=CH\text{-}P\emptyset_2$

d) $\emptyset_2As\text{-}CH=CH\text{-}As\emptyset_2$

e) $\emptyset_2P\text{-}CH_2\text{-}CH_2\text{-}P\emptyset\text{-}CH_2\text{-}CH_2\text{-}P\emptyset\text{-}CH_2CH_2\text{-}P\emptyset_2$

f) $\emptyset_2P\text{-}CH_2\text{-}CH_2\text{-}P\emptyset\text{-}CH_2\text{-}CH_2\text{-}P\emptyset_2$

g)

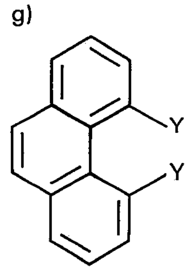

h) $P(\text{-}CH_2CH_2\text{-}P\emptyset_2)_3$

i) $R^1\text{-}C[\text{-}(CH_2)_n\text{-}Y]_3$

j)

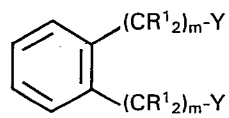

k) $\emptyset_2P\text{-}(CH_2)_x$ / $\emptyset_2P\text{-}(CH_2)_x$ >N-$CH_2$-$CH_2$-N< $(CH_2)_x\text{-}P\emptyset_2$ / $(CH_2)_x\text{-}P\emptyset_2$

wobei

$\emptyset = C_6H_5\text{-}$

Y = $\text{-}NR^2_2$, ein stickstoffhaltiger Arylrest, $\text{-}PR^2_2$, $\text{-}AsR^2_2$, $\text{-}SR^2$ oder -SH;

$R^1$ = -H, $C_1$ bis $C_5$-Alkyl oder $\text{-}C_6H_5$;

$R^2$ = $C_1$ bis $C_6$-Alkyl, $C_5$ bis $C_8$ Cycloalkyl oder $\text{-}C_6H_5$ oder $C_6H_5CH_2\text{-}$, die ggf. mit Halogen-, Methoxy-, Ethoxy- oder $C_1$ bis $C_3$-Alkyl substituiert sind;

n = 1-6, vorzugsweise 1-4;

m = 0-8, vorzugsweise 0-3;

x = 1 oder 2.

5. Verfahren nach einem der Ansprüche 1-4, dadurch gekennzeichnet, dass der Trägerkatalysator ein anorganisches oxidisches Trägermaterial oder einen Aktivkohleträger enthält.

6. Verfahren nach einem der Ansprüche 1-5, dadurch gekennzeichnet, dass der Trägerkatalysator zusammen 0,01 bis 50 Gew.-% vorzugsweise 0,1 bis 20 Gew.-%, Chelatverbindungen und ggf. Nichtedelmetallverbindungen enthält.

7. Verfahren nach einem der Ansprüche 1-6, dadurch gekennzeichnet, dass der Trägerkatalysator in einer Korngrösse von 1 bis 20 mm eingesetzt wird.

8. Trägerkatalysator für die Herstellung von Monocarbonsäureanhydriden durch Carbonylierung der entsprechenden Ester oder Ether, dadurch gekennzeichnet, dass in ihm das Trägermaterial eine Edelmetall-Chelatverbindung trägt, die aus einer Edelmetallverbindung der 8. Nebengruppe des Periodensystems der Elemente und einem Chelator mit Organostickstoff-, Organophosphor-, Organoarsen- oder Organoschwefelgruppen gebildet ist.

9. Trägerkatalysator nach Anspruch 8, dadurch gekennzeichnet, dass in ihm das Trägermaterial zusätzlich eine Nichtedelmetall-Chelatverbindung trägt, die aus einer Nichtedelmetallverbindung aus der 6. oder 8. Nebengruppe des Periodensystems der Elemente und einem Chelator mit Organostickstoff-, Organophosphor-, Organoarsen- oder Organoschwefelgruppen gebildet ist.

10. Trägerkatalysator nach Anspruch 8 oder 9, dadurch gekennzeichnet, dass er zusätzlich Nichtedelmetallverbindungen aus der 1. bis 3. Hauptgruppe oder der 4. bis 6. oder der 8. Nebengruppe des Periodensystems der Elemente als Promotoren enthält.

11. Trägerkatalysator nach einem der Ansprüche 8 bis 10, dadurch gekennzeichnet, dass in ihm das Trägermaterial eine Chelatverbindung trägt, die aus der Metallverbindung und einem der folgenden Chelatoren gebildet ist:

a) $Y-(CH_2)_n-Y$

b) $Y-CH=CH-Y$

c) $Ø_2P-CH=CH-PØ_2$

d) $Ø_2As-CH=CH-AsØ_2$

e) $Ø_2P-CH_2-CH_2-PØ-CH_2-CH_2-PØ-CH_2CH_2-PØ_2$

f) $Ø_2P-CH_2-CH_2-PØ-CH_2-CH_2-PØ_2$

g)

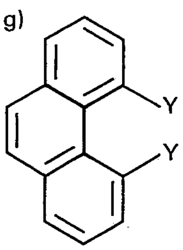

h) $P(-CH_2CH_2-PØ_2)_3$

i) $R^1-C[-(CH_2)_n-Y]_3$

j)

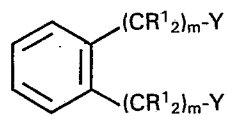

k) $Ø_2P-(CH_2)_x$, $Ø_2P-(CH_2)_x$ >N-$CH_2$-$CH_2$-N< $(CH_2)_x-PØ_2$, $(CH_2)_x-PØ_2$

wobei

$Ø = C_6H_5-$

$Y = -NR^2_2$, ein stickstoffhaltiger Arylrest, $-PR^2_2$, $-AsR^2_2$, $-SR^2$ oder $-SH$;

$R^1 = -H$, $C_1$ bis $C_5$-Alkyl oder $-C_6H_5$;

$R^2 = C_1$ bis $C_6$-Alkyl, $C_5$ bis $C_8$ Cycloalkyl oder $-C_6H_5$ oder $C_6H_5CH_2-$, die ggf. mit Halogen-, Methoxy-, Ethoxy- oder $C_1$ bis $C_3$-Alkyl substituiert sind;

n = 1-6, vorzugsweise 1-4;

m = 0-8, vorzugsweise 0-3;

x = 1 oder 2.

12. Trägerkatalysator nach einem der Ansprüche 8-11, dadurch gekennzeichnet, dass er ein anorganisches oxidisches Trägermaterial oder einen Aktivkohleträger enthält.

13. Trägerkatalysator nach einem der Ansprüche 8-12, dadurch gekennzeichnet, dass er zusammen 0,01 bis 50 Gew.-%, vorzugsweise 0,1 bis 20 Gew.-%, Chelatverbindungen und ggf. Nichtedelmetallverbindungen enthält.

14. Trägerkatalysator nach einem der Ansprüche 8-13, gekennzeichnet durch die Formel

Träger ] $[Rh([C_6H_5]_2P-CH_2-CH_2-P[C_6H_5]_2)_2]Cl$.

15. Trägerkatalysator nach einem der Ansprüche 8-13, gekennzeichnet durch die Formel

Träger ] $[Rh([C_6H_5]_2P-CH_2-CH_2-P[C_6H_5]_2)_2]BF_4$.

16. Trägerkatalysator nach einem der Ansprüche 8-13, gekennzeichnet durch die Formel

Träger ] $[Rh([C_6H_5]_2P-[CH_2]_4-P[C_6H_5]_2)(CO)Cl]_2$.

17. Trägerkatalysator nach einem der Ansprüche 8-13, gekennzeichnet durch die Formel

Träger ] $[Rh([C_6H_5]_2PCH_2CH_2P[C_6H_5]CH_2CH_2P[C_6H_5]CH_2CH_2P[C_6H_5]_2)]PF_6$.

18. Trägerkatalysator nach einem der Ansprüche 8-13, gekennzeichnet durch die Formel

Träger ] $[Rh([C_6H_5]_2P-CH_2-CH_2-P[C_6H_5]_2)_2]BF_4$
$[Cr(C_6H_5)_2P-CH_2-CH_2-P(C_6H_5)_2(CO)_4]$.

19. Trägerkatalysator nach einem der Ansprüche 8-13, gekennzeichnet durch die Formel

Träger ] $[Rh([C_6H_5]_2P-CH_2-CH_2-P[C_2H_5]_2)_2]BF_4$
NaJ.

20. Trägerkatalysator nach einem der Ansprüche 8-13, gekennzeichnet durch die Formel

Träger ] $[Rh([C_6H_5]_2P-CH=CH-P[C_6H_5]_2)_2]ClO_4$.

**Claims**

1. A process for the preparation of a monocarboxylic anhydride of the formula $(RCO)_2O$ by reacting a carboxylate or dialkyl ether of the formula RCOOR or ROR respectively, where R in each case denotes the same alkyl radical having 1-4 carbon atoms, with carbon monoxide in the gas phase in the presence of iodine or bromine or compounds thereof as reaction promoters, and in the presence of a supported catalyst containing noble-metal compounds from group VIII of the Periodic Table, at a temperature from 130 to 400°C and at a pressure from 1-150 bar, wherein the support material in the supported catalyst carries a noble-metal chelate compound which is formed from the noble-metal compound and a chelating agent containing organonitrogen, organophosphorus, organoarsenic or organosulfur groups.

2. The process as claimed in claim 1, wherein the support material in the supported catalyst additionally carries a non noble-metal chelate compound which is formed from a non noble-metal compound from sub-group 6 or 8 of the Periodic Table of the Elements and a chelating agent containing organonitrogen, organophosphorus, organoarsenic or organosulfur groups.

3. The process as claimed in claim 1 or 2, wherein the supported catalyst additionally contains, as promoters, non noble-metal compounds from main groups 1 to 3 or sub-groups 4 to 6 or 8 of the Periodic Table of the Elements.

4. The process as claimed in one of claims 1 to 3, wherein the support material in the supported catalyst carries a chelate compound which is formed from the metal compound and one of the following chelating agents:

a) $Y-(CH_2)_n-Y$

b) $Y-CH=CH-Y$

c) $\emptyset_2P-CH=CH-P\emptyset_2$

d) $\emptyset_2As-CH=CH-As\emptyset_2$

e) $\emptyset_2P-CH_2-CH_2-P\emptyset-CH_2-CH_2-P\emptyset-CH_2CH_2-P\emptyset_2$

f) $\emptyset_2P-CH_2-CH_2-P\emptyset-CH_2-CH_2-P\emptyset_2$

g)

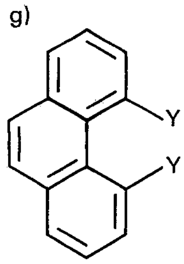

h) $P(-CH_2CH_2-P\emptyset_2)_3$

i) $R^1-C[-(CH_2)_n-Y]_3$

j)

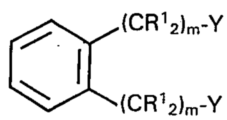

k) $\emptyset_2P-(CH_2)_x$ $(CH_2)_x-P\emptyset_2$
$\emptyset_2P-(CH_2)_x$ >N-$CH_2-CH_2$-N< $(CH_2)_x-P\emptyset_2$

where
$\emptyset = C_6H_5-$

Y = $-NR^2_2$, a nitrogen-containing aryl radical, $-PR^2_2$, $-AsR^2_2$, $-SR^2$ or -SH;

$R^1$ = -H, $C_1$ to $C_5$-alkyl or $-C_6H_5$;

$R^2$ = $C_1$ to $C_6$-alkyl, $C_5$ to $C_8$-cycloalkyl or $-C_6H_5$ or $C_6H_5CH_2-$ which are optionally substituted by halogen-, methoxy-, ethoxy- or $C_1$ to $C_3$-alkyl;

n = 1-6, preferably 1-4;

m = 0-8, preferably 0-3;

x = 1 or 2.

5. The process as claimed in one of claims 1-4, wherein the supported catalyst contains an inorganic oxidic support material or an activated charcoal support.

6. The process as claimed in one of claims 1-5, wherein the supported catalyst contains in total 0.01 to 50% by weight, preferably 0.1 to 20% by weight of chelate compounds, and, where appropriate, non noble-metal compounds.

7. The process as claimed in one of claims 1-6, wherein the supported catalyst is employed in a grain size of 1 to 20 mm.

8. A supported catalyst for the preparation of a monocarboxylic anhydride by carbonylation of the appropriate ester or ether, wherein the support material in the supported catalyst carries a noble metal chelate compound which is formed from a noble-metal compound of sub-group 8 of the Periodic Table of the Elements and a chelating agent containing organonitrogen, organophosphorus, organoarsenic or organosulfur groups.

9. A supported catalyst as claimed in claim 8,

wherein the support material in the supported catalyst additionally carries a non noble-metal chelate compound which is formed from a non noble-metal compound from sub-group 6 or 8 of the Periodic Table of the Elements and a chelating agent containing organonitrogen, organophosphorus, organoarsenic or organosulfur groups.

10. A supported catalyst as claimed in claim 8 or 9, which additionally contains, as promoters, non noble-metal compounds from main groups 1 to 3, or sub-groups 4 to 6 or 8 of the Periodic Table of the Elements.

11. A supported catalyst as claimed in claim 8 to 10, wherein the support material in the supported catalyst carries a chelate compound which is formed from the metal compound and one of the following chelating agents:

a) $Y-(CH_2)_n-Y$

b) $Y-CH=CH-Y$

c) $\emptyset_2P-CH=CH-P\emptyset_2$

d) $\emptyset_2As-CH=CH-As\emptyset_2$

e) $\emptyset_2P-CH_2-CH_2-P\emptyset-CH_2-CH_2-P\emptyset-CH_2CH_2-P\emptyset_2$

f) $\emptyset_2P-CH_2-CH_2-P\emptyset-CH_2-CH_2-P\emptyset_2$

g)

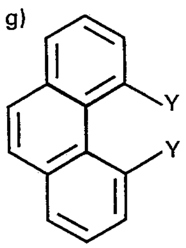

h) $P(-CH_2CH_2-P\emptyset_2)_3$

i) $R^1-C[-(CH_2)_n-Y]_3$

j)

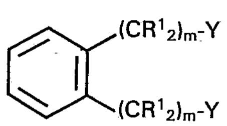

k) $\emptyset_2P-(CH_2)x$ $(CH_2)_x-P\emptyset_2$
$\emptyset_2P-(CH_2)_x$ >N-CH₂-CH₂-N< $(CH_2)_x-P\emptyset_2$

where
$\emptyset = C_6H_5-$

$Y = -NR^2_2$, a nitrogen-containing aryl radical, $-PR^2_2$, $-AsR^2_2$, $-SR^2$ or $-SH$;

$R^1 = -H$, $C_1$ to $C_5$-alkyl or $-C_6H_5$;

$R^2 = C_1$ to $C_6$-alkyl, $C_5$ to $C_8$ cycloalkyl or $-C_6H_5$ or $C_6H_5CH_2$- which are optionally substituted by halogen-, methoxy-, ethoxy- or $C_1$ to $C_3$-alkyl;

n = 1-6, preferably 1-4;

m = 0-8, preferably 0-3;

x = 1 or 2.

12. A supported catalyst as claimed in one of claims 8-11, which contains an inorganic oxidic support material or an activated charcoal support.

13. A supported catalyst as claimed in one of claims 8-12, which contains in total 0.01 to 50% by weight, preferably 0.1 to 20% by weight, of chelate compounds and, where appropriate, non noble-metal compounds.

14. A supported catalyst as claimed in one of claims 8-13, which has the formula

support ] $[Rh([C_6H_5]_2P-CH_2-CH_2-P[C_6H_5]_2)_2]Cl$.

15. A supported catalyst as claimed in one of claims 8-13, which has the formula

support ] $[Rh([C_6H_5]_2P-CH_2-CH_2-P[C_6H_5]_2)_2]BF_4$.

16. A supported catalyst as claimed in one of claims 8-13, which has the formula

support ] $[Rh([C_6H_5]_2P-[CH_2]_4-P[C_6H_5]_2)(CO)Cl]_2$.

17. A supported catalyst as claimed in one of claims 8-13, which has the formula

support ] $[Rh([C_6H_5]_2PCH_2CH_2P[C_6H_5]CH_2CH_2P$ $[C_6H_5]\ CH_2CH_2P[C_6H_5]_2)]PF_6$.

18. A supported catalyst as claimed in one of claims 8-13, which has the formula

support ] $[Rh([C_6H_5]_2P-CH_2-CH_2-P[C_6H_5]_2)_2]BF_4$.
$[Cr(C_6H_5)_2P-CH_2-CH_2-P(C_6H_5)_2(CO)_4]$.

19. A supported catalyst as claimed in one of claims 8-13, which has the formula

support ] $[Rh([C_6H_5]_2P-CH_2-CH_2-P[C_2H_5]_2)_2]BF_4$
NaI.

20. A supported catalyst as claimed in one of claims 8-13, which has the formula

support ] $[Rh([C_6H_5]_2P-CH=CH-P[C_6H_5]_2)_2]ClO_4$.

**Revendications**

1. Procédé de préparation d'anhydrides d'acides monocarboxyliques de formule générale $(RCO)_2O$ par réaction d'un ester d'acide carboxylique ou d'un éther dialkylique de formules générales respectives RCOOR et ROR, dans lesquelles R représente dans chaque cas le même groupe alkyle en $C_1$-$C_4$, avec l'oxyde de carbone en phase gazeuse en présence

d'iode ou de brome ou de leurs composés servant d'activateurs de reaction et en présence d'un catalyseur sur support contenant des composés de métaux nobles du groupe VIII de la classification périodique à des températures de 130 à 400°C et des pressions de 1 à 150 bar, caractérisé en ce que, dans le catalyseur sur support, la matière de support porte un chélate de métal noble formé à partir du composé de métal noble et d'un agent chélatant contenant des groupes organo-azotés, organo-phosphorés, organo-arséniés ou organo-soufrés.

2. Procédé selon la revendication 1, caractérisé en ce que, dans le catalyseur sur support, la matière de support porte en outre un chélate de métal non noble formé à partir d'un composé de métal non noble du 6e ou du 8e sous-groupe de la classification périodique des éléments et d'un agent chélatant contenant des groupes organo-azotés, organo-phosphorés, organo-arséniés ou organo-soufrés.

3. Procédé selon la revendication 1 ou 2, caractérisé en ce que le catalyseur sur support contient en outre des composés de métaux non nobles du 1er au 3e groupe principal ou du 4e au 6e ou du 8e sous-groupe de la classification périodique des éléments en tant qu'activateurs.

4. Procédé selon l'une des revendications 1 à 3, caractérisé en ce que, dans le catalyseur sur support, la matière de support porte un chélate formé à partir du composé métallique et de l'un des agents chélatants suivants:

a) $Y-(CH_2)_n-Y$

b) $Y-CH=CH-Y$

c) $\emptyset_2P-CH=CH-P\emptyset_2$

d) $\emptyset_2As-CH=CH-As\emptyset_2$

e) $\emptyset_2P-CH_2-CH_2-P\emptyset-CH_2-CH_2-P\emptyset-CH_2CH_2-P\emptyset_2$

f) $\emptyset_2P-CH_2-CH_2-P\emptyset-CH_2-CH_2-P\emptyset_2$

g)

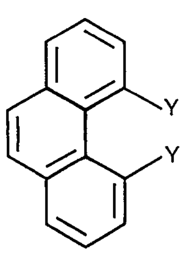

h) $P(-CH_2CH_2-P\emptyset_2)_3$

i) $R^1-C[-(CH_2)_n-Y]_3$

j)

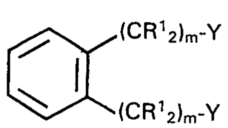

k) $\emptyset_2P-(CH_2)x$ $(CH_2)_x-P\emptyset_2$
$\emptyset_2P-(CH_2)_x$ >N-$CH_2-CH_2$-N< $(CH_2)_x-P\emptyset_2$

dans lesquels
$\emptyset = C_6H_5-$

Y = $-NR^2_2$, un groupe aryle contenant de l'azote, $-PR^2_2$, $-AsR^2_2$, $-SR^2$ ou -SH;

$R^1$ = -H, alkyle en $C_1$ - $C_5$ ou $-C_6H_5$;

$R^2$ = alkyle en $C_1$ - $C_6$, cycloalkyle en $C_5$ à $C_8$ ou $-C_6H_5$ ou $C_6H_5CH_2-$ éventuellement substitués par des halogènes, des groupes méthoxy, éthoxy ou alkyle en $C_1$ - $C_3$;

n = 1 à 6, de préférence 1 à 4;

m = 0 à 8, de préférence 0 à 3;

x = 1 ou 2.

5. Procédé selon l'une des revendications 1 à 4, caractérisé en ce que le catalyseur sur support contient une matière de support du type oxyde inorganique ou un support du type charbon actif.

6. Procédé selon l'une des revendications 1 à 5, caractérisé en ce que le catalyseur sur support contient au total de 0,01 à 50% en poids, de préférence de 0,1 à 20% en poids, de chélates et, le cas échéant, de composés de métaux non nobles.

7. Procédé selon l'une des revendications 1 à 6, caractérisé en ce que le catalyseur sur support est mis en oeuvre à une dimension de grain de 1 à 20 mm.

8. Catalyseur sur support pour la préparation d'anhydrides d'acides monocarboxyliques par carbonylation des esters ou éthers correspondants, caractérisé en ce que, dans ce catalyseur sur support, la matière de support porte une chélate de métal noble formé à partir d'un composé de métal noble du 8e sous-groupe de la classification périodique des éléments et d'un agent chélatant contenant des groupes organo-azotés, organo-phosphorés, organo-arséniés ou organo-soufrés.

9. Catalyseur sur support selon la revendication 8, caractérisé en ce que, dans ce catalyseur sur support, la matière de support porte en outre un chélate de métal non noble formé à partir d'un composé de métal non noble du 6e ou du 8e sous-groupe de la classification périodique des éléments et d'un agent chélatant contenant des groupes organo-azotés, organo-phosphorés, organo-arséniés ou organo-soufrés.

10. Catalyseur sur support selon la revendication 8 ou 9, caractérisé en ce qu'il contient en outre des composés de métaux non nobles du 1er au 3e groupe principal ou du 4e au 6e ou du 8e sous-groupe de la classification périodique des éléments en tant qu'activateurs.

11. Catalyseur sur support selon l'une des revendications 8 à 10, caractérisé en ce que, dans ce catalyseur sur support, la matière de support porte un chélate formé à partir du composé métallique et de l'un des agents chélatants suivants:

a) $Y-(CH_2)_n-Y$

b) $Y-CH=CH-Y$

c) $\emptyset_2P-CH=CH-P\emptyset_2$

d) $\emptyset_2As-CH=CH-As\emptyset_2$

e) $\emptyset_2P-CH_2-CH_2-P\emptyset-CH_2-CH_2-P\emptyset-CH_2CH_2-P\emptyset_2$

f) $\emptyset_2P-CH_2-CH_2-P\emptyset-CH_2-CH_2-P\emptyset_2$

g)

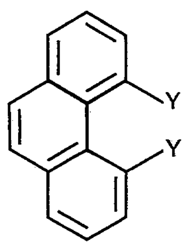

h) $P(-CH_2CH_2-P\emptyset_2)_3$

i) $R^1-C[-(CH_2)_n-Y]_3$

j)

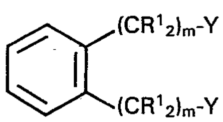

k) $\emptyset_2P-(CH_2)x$ $(CH_2)_x-P\emptyset_2$
$\emptyset_2P-(CH_2)_x$ $>N-CH_2-CH_2-N<$ $(CH_2)_x-P\emptyset_2$

dans lesquels
$\emptyset = C_6H_5-$

$Y = -NR^2_2$, un groupe aryle contenant de l'azote, $-PR^2_2$, $-AsR^2_2$, $-SR^2$ ou $-SH$;

$R^1 = -H$, alkyle en $C_1$ - $C_5$ ou $-C_6H_5$;

$R^2$ = alkyle en $C_1$ - $C_6$, cycloalkyle en $C_5$ à $C_8$ ou $-C_6H_5$ ou $C_6H_5CH_2$- éventuellement substitués par des halogènes, des groupes méthoxy, éthoxy ou alkyle en $C_1$ - $C_3$;

n = 1 à 6, de préférence 1 à 4;

m = 0 à 8, de préférence 0 à 3;

x = 1 ou 2.

12. Catalyseur sur support selon l'une des revendications 8 à 11, caractérisé en ce qu'il contient une matière de support du type oxyde inorganique ou un support du type charbon actif.

13. Catalyseur sur support selon l'une des revendications 8 à 12, caractérisé en ce qu'il contient au total de 0,01 à 50% en poids, de préférence de 0,1 à 20% en poids de chélates et, le cas échéant, de composés de métaux non nobles.

14. Catalyseur sur support selon l'une des revendications 8 à 13, caractérisé par la formule

Support ] $[Rh([C_6H_5]_2P-CH_2-CH_2-P[C_6H_5]_2)_2]Cl$.

15. Catalyseur sur support selon l'une des revendications 8 à 13, caractérisé par la formule

Support ] $[Rh([C_6H_5]_2P-CH_2-CH_2-P[C_6H_5]_2)_2]BF_4$.

16. Catalyseur sur support selon l'une des revendications 8 à 13, caractérisé par la formule

Support ] $[Rh([C_6H_5]_2P-[CH_2]_4-P[C_6H_5]_2)(CO)Cl]_2$.

17. Catalyseur sur support selon l'une des revendications 8 à 13, caractérisé par la formule

Support ] $[Rh([C_6H_5]_2PCH_2CH_2P[C_6H_5]CH_2CH_2P$
$[C_6H_5]\ CH_2CH_2P[C_6H_5]_2)]PF_6$.

18. Catalyseur sur support selon l'une des revendications 8 à 13, caractérisé par la formule

Support ] $[Rh([C_6H_5]_2P-CH_2-CH_2-P[C_6H_5]_2)_2]BF_4$
$[Cr(C_6H_5)_2P-CH_2-CH_2-P(C_6H_5)_2(CO)_4]$.

19. Catalyseur sur support selon l'une des revendications 8 à 13, caractérisé par la formule

Support ] $[Rh([C_6H_5]_2P-CH_2-CH_2-P[C_2H_5]_2)_2]BF_4$
NaI.

20. Catalyseur sur support selon l'une des revendications 8 à 13, caractérisé par la formule

Support ] $[Rh([C_6H_5]_2P-CH=CH-P[C_6H_5]_2)_2]ClO_4$.